# EUROPEAN PATENT SPECIFICATION

(11) **EP 2 404 623 B1**
(45) Date of publication and mention of the grant of the patent: **10.01.2018**
(21) Application number: 10748838.9
(22) Date of filing: 05.03.2010
(51) Int. Cl.: A61L 27/00, A61L 27/36

(54) **BONE TISSUE REGENERATION WITH CALCIFIED SUBSTANCE PRODUCED BY CULTURED CELLS**
KNOCHENREGENERATION MITTELS EINES VON KULTURZELLEN PRODUZIERTEN KALKSTOFFES
RÉGÉNÉRATION D'OS AU MOYEN D'UNE SUBSTANCE CALCIFIÉE PRODUITE PAR CULTURE DE CELLULES

(30) Priority: 06.03.2009 JP 2009054261
(43) Date of publication of application: 11.01.2012
(73) Proprietor: Niigata University, Niigata 950-2181 (JP)
(72) Inventor: KAWASE, Tomoyuki, Niigata-shi Niigata 951-8514 (JP)
(74) Representative: Beetz & Partner mbB
(86) International application number: PCT/JP2010/053661
(87) International publication number: WO 2010/101245

(56) References cited:
- WO-A1-2008/035843
- WO-A1-2009/025374
- JP-A- 2002 078 791
- JP-A- 2004 305 260
- JP-A- 2008 183 324
- JP-T- 8 510 984
- US-A1- 2002 013 626
- LIISA T. KUHN ET AL: "Structure, Composition, and Maturation of Newly Deposited Calcium-Phosphate Crystals in Chicken Osteoblast Cell Cultures", JOURNAL OF BONE AND MINERAL RESEARCH, vol. 15, no. 7, 1 July 2000 (2000-07-01), pages 1301-1309, XP055105899, ISSN: 0884-0431, DOI: 10.1359/jbmr.2000.15.7.1301
- "Medical Devices: Guidance document", , 3 December 2009 (2009-12-03), XP055023202, Retrieved from the Internet: URL:http://ec.europa.eu/health/medical-dev ices/files/meddev/2_1_3_rev_3-12_2009_en.p df [retrieved on 2012-03-28]

## Description

### TECHNICAL FIELD

The present invention relates to the field of a material for the purpose of regenerating a bone tissue and specifically to a method for producing a base material according to claim 1.

### BACKGROUND ART

There have been many kinds of materials for the purpose of regeneration of bone tissues, and the materials, which possess not only the osteoconductive potential, but also osteoinductive potential, have been actively developed recently. Ceramic materials made of hydroxyapatite (HAp), a main inorganic ingredient of the bone and one of the most frequently used for clinical application, can be produced to high rigidity due to sintering at a high temperature, although high absorbability in the body cannot be anticipated. While their accelerating effects on osteoconduction have often been reported, there has been no report regarding induction of calcification after subcutaneous implantation possibly due to less absorbability in the body.
On the other hand, ceramic materials made of calcium phosphate, as represented by α (alpha)-TCP and β (beta)-TCP, are absorbed by osteoclasts in a comparatively high rate when implanted into the body. Furthermore, when an osteoblastic cell is present nearby, the decomposed calcium (Ca) and phosphorus (P) are readily utilized to form a bone. However, when the ceramic materials made of calcium phosphate are supplemented to the osteoblastic cell or periosteal sheet in vitro, they cannot induce calcification due to lack of osteoclastic cell. Accordingly, β (beta)-TCP can be considered to possess indirect ability to induce a bone.

The inventors developed a technique to shorten the culture period by consideration on methods for collecting and cultivating human periosteum (Patent Document 1). However, artificial materials such as HAp, α (alpha)-TCP and β (beta)-TCP, etc., do not accelerate the calcification potential of cultured periosteum.

In contrast to the artificial materials such as HAp, α (alpha)-TCP and β (beta)-TCP, etc., cells can produce calcified substance. For example, it is known that a sea urchin larva has a skeletone with a species-specific fine structure, which is known to be formed by a syncytium of a few dozens of cells belonging to a particular cell lineage in the early embryo belonging to specific cell genealogy of echinus nascent embryo cells (Non-Patent Document 1). Therefore, it is speculated that, even in the species of the mammals including the human, the calcified substances produced by osteoblastic cells are readily incorporated into existing hard tissues, and that the calcified substances have suitable structures for degradation by osteoclastic cells and recycle by osteoblastic cells.

### PRIOR-ART DOCUMENTS

### Patent Document

Patent Document 1. WO2009/025374 pamphlet

### Non-Patent Document

Non-Patent Document 1. Okazaki, K. and Inoue, S., Develop. Growth and Deffer., 18:413 (1976)

### SUMMARY OF INVENTION

### PROBLEM TO BE SOLVED BY THE PRESENT INVENTION

There has been a need to develop a material, which possesses a higher and more direct bone-induction ability, by utilizing a calcified substance produced by a cell.

### MEANS FOR SOLVING THE PROBLEM

The present invention provides a method for producing a base material for regenerating a bone tissue, which comprises a calcified substance extracted from a cultured cell expressing calcification capacity.
In the material of the present invention for regenerating a bone tissue, the said cultured cell expressing the calcification capacity may be a mammalian cell.
In the material of the present invention for regenerating a bone tissue, the said cultured cell expressing the calcification capacity may be a human cell.
In the material of the present invention for regenerating a bone tissue, the said cultured cell expressing the calcification capacity may be a cell derived from a hard tissue.
In the material of the present invention for regenerating a bone tissue, the said cultured cell expressing the calcification capacity may be a cell derived from an induced pluripotent stem cell.

In the material of the present invention for regenerating a bone tissue, the said cultured cell expressing the calcification capacity may be a cell derived from a mesenchymal stem cell.
In the material of the present invention for regenerating a bone tissue, the said cultured cell expressing the calcification capacity may be a cell derived from an osteosarcoma. In the material of the present invention for regenerating a bone tissue, the said calcified substance may be extracted without sintering at a high temperature. In the material of the present invention for regenerating a bone tissue, the said calcified substance may be extracted by use of a detergent. Described herein is an artificial bone comprising the material of the present invention for regenerating a bone tissue Described herein is a method for enhancing the calcification capacity of a hard tissue cell, comprising a step of supplementing the material of the present invention for regenerating a bone tissue. Described herein is a bone filler comprising the material for regenerating a bone tissue of the present invention, and a cell with the calcification capacity. The present invention provides a method for producing the material for regenerating a bone tissue, comprising the said calcified substance as disclosed in claim 1.

In the said method to produce the material for regenerating a bone tissue of the present invention, the cultured cell may be a mammalian cell.
In the said method to produce the material for regenerating a bone tissue of the invention, the cultured cell may be a human cell.

In the method to produce the material for regenerating a bone tissue of the present invention, the said cultured cell expressing a calcification capacity may be derived from a hard tissue.

In the method for producing the material regenerating a bone tissue of the present invention, the said cultured cell with the calcification capacity may be derived from an induced pluripotent stem cell.

In the method for producing the material of the present invention for regenerating a bone tissue, the said cultured cell with the calcification capacity may be derived from a mesenchymal stem cell.

In the method for producing the material of the present invention for regenerating a bone tissue, the said cultured cell with a calcification capacity may be derived from an osteosarcoma cell.

In the method for producing the material of the present invention for regenerating a bone tissue, the step of extracting the calcified substance may comprise extracting calcified substance without sintering at a high temperature.

In the method for producing the material of the present invention for regenerating a bone tissue, the step of extracting a calcified substance may comprise extracting the said calcified substance by use of a detergent.

### BRIEF DESCRIPTION OF THE DRAWINGS

[Fig. 1A] A microscopic photograph of a calcified substance stained with alizarin red in a UMR-106 cell cultured for five days in a culture dish.
[Fig.1B] An electronic scanning microscopic photograph of the calcified substance in a UMR-106 cell in a culture dish cultured for five days after switching to differentiation medium.
[Fig. 2A] An electronic scanning microscopic photograph of the calcified substance extracted from UMR-106 cells in a culture dish cultured for five days after switching to differentiation medium: Low magnification.
[Fig. 2B] An electronic scanning microscopic photograph of the calcified substance extracted from human periosteal cells in a culture dish cultured for 24 days after switching to differentiation medium: Low magnification.
[Fig. 2C] An electronic scanning microscopic photograph of a calcified substance extracted from UMR-106 cells in a culture dish cultured for five days: High magnification.
[Fig. 2D] An electronic scanning microscopic photograph of the calcified substance extracted from the human periosteal cells in a culture dish cultured for 24 days after switching to differentiation medium: High magnification.
[Fig. 3A] A Fourier transform infrared spectra of calcified substance sample extracted from UMR-106 cells and HAp.
[Fig. 3B] A Fourier transform infrared spectra of calcified substance sample extracted from UMR-106 cells and β (beta)-TCP.
[Fig. 4] X-ray diffraction spectra of calcified substance sample extracted from UMR-106 cells, calcified substance sample from cultured human periosteal cells, HAp and β (beta)-TCP.
[Fig. 5A] A microscopic photograph of a human periosteal sheet stained with HE (hematoxilyn and eosin). An undifferentiated human periosteal cell sheet was supplemented with calcified substance sample extracted from UMR-106 cells and further cultured for eight days.
[Fig. 5B] A microscopic photograph of a human periosteal sheet stained with von Kossa staining. An undifferentiated human periosteal cell sheet was supplemented with calcified substance sample extracted from UMR-106 cells and further cultured for eight days.
[Fig. 6A] A microscopic photograph of a human periosteal sheet stained with HE (hematoxilyn and eosin). A differentiated human periosteal cell sheet was supplemented with calcified substance sample extracted from UMR-106 cells and further cultured for eight days.
[Fig. 6B] A microscopic photograph of a human periosteal sheet stained with von Kossa staining. A differentiated human periosteal cell sheet was supplemented with calcified substance sample extracted from UMR-106 cells and further cultured for eight days.
[Fig. 7A] A microscopic photograph of a human periosteal sheet stained with HE (hematoxilyn and eosin). A differentiated human periosteal cell sheet was supplemented with pulverized β (beta)-TCP particles and further cultured for eight days.
[Fig. 7B] A microscopic photograph of a human periosteal sheet stained with von Kossa staining. A differentiated human periosteal cell sheet was supplemented with pulverized β (beta)-TCP particles and further cultured for eight days.
[Fig. 8] A µ (micro)-CT image of a nude mouse one week after implantation of a differentiated periosteal cell sheet. A differentiated human periosteal cell sheet was supplemented with calcified substance sample extracted from UMR-106 cells, and implanted under bilateral back skin region.
[Fig. 9A] A microscopic photograph of a HE-stained section of a differentiated periosteal cell sheet, which had been cultured for eight days after supplementing the calcified substance sample extracted from UMR-106 cells and implanted under bilateral back skin region.
[Fig. 9B] A microscopic photograph of a von Kossa-stained differentiated periosteal cell sheet, which had been cultured for eight days after supplementing the calcified substance sample extracted from UMR-106 cells and implanted under bilateral back skin region.

### DESCRIPTION OF EMBODIMENTS

In the present invention, a calcified substance is a substance comprising calcium phosphate as a main ingredient produced from hard tissues including bone and teeth. A calcification capacity is defined as an ability to produce the said calcified substance.

In the present specification, a cell having the calcification capacity is included in hard tissue cells in the body, but not limited to osteoblastic cell, bone-forming cell, odontoblast, ameloblast, or cementoblast. A cultured cell having calcification capacity in the present invention is defined as a cell derived from either a cell belonging to a cell lineage common to the hard tissue cells in the body, or a cell which proliferate under a culture condition, transformed or transdifferentiated to a cell expressing genes specific to the hard tissue cells in the body.

The cultured cell having the calcification capacity of the present invention is required not to express constitutive calcification capacity under the regular culture condition, but to include a cell which expresses detectable calcification capacity after it is cultured in a differentiation-inducing medium; this cell, barely or not at all, expresses the calcification capacity while culturing in a growth medium. A procedure for the differentiation-induction is not limited to a single step, but could be expanded to two or more steps, as described in the examples of the present specification.

An example of differentiation induced by two steps is a case where differentiation is induced in a cell, such as a mesenchymal stem cell that can initially differentiate to cell types (e.g. muscle, cartilage, etc.) distinguishable from cells of a hard tissue expressing calcification capacity, and secondary differentiate to produce a calcified substance.

An example of three-step induction of differentiation is a case where the above-mentioned two-step induction is preceded by a step of inducing a pluripotential cell to differentiate into a cell which can be differentiated only to a mesenchymal stem cell; the pluripotential cell being induced pluripotent cell, an embryo stem cell (ES cell), a stem cell in the body, etc., which can differentiate into a non-mesenchymal cell type such as many cell types constituting a body including neuron, sensory organ, epidermis, intestinal epithelia, blood cells, endocrine gland, exocrine gland, and reproductive gland.

In addition, culturing in the differentiation medium of the present invention involves a case where differentiation of the pluripotent cell capable of differentiating to the cells distinguishable from mesenchymal type is induced directly to the cell expressing a calcification capacity.

Furthermore, the cultured cell having calcification capacity of the present invention includes not only a normal cell in the body, but also a cell that is derived from a benign or malignant tumor or a cancer cell relating to a hard tissue, including, but not limited to, an osteosarcoma cell, and is capable of proliferating under culture conditions

In the present specification, the condition for inducing cell differentiating includes, but not limited to, supplementation or intracellular delivery of a low molecular substance such as dexamethasone, β (beta)-glycerophosphoric acid, ascorbic acid, retinoic acid, 5-azacitidine, or valproic acid, etc., supplementation or cellular delivery of a protein or peptide which includes a cytokine such as basic fibroblast growth factor, transforming growth factor-β (beta), etc., and a transcription factor such as Oct4, Sox2, c-Myc, Klf4, MyoD etc., and a protein or peptide which associates specifically to their receptors; a protein or peptide which can specifically bind to them; supplementation or intracellular delivery of nucleic acid such as a polynucleotide or oligonucleotide, etc., which can regulate the expression of a gene encoding the protein or peptide; supplementation or intracellular delivery of a vector such as a virus plasmid, etc., which comprises the nucleotide thereof; use of 2- or 3- dimensional scaffold or architecture for cells; and, use of shaking culture, bead-shaped substrate.

Extracting a calcified substance of the present invention means removing organic substances which are derived from a culture medium and a cell, from calcified substances produced by cells. Extracting the calcified substances may involve procedures for evaporating water by heating and for inactivating a microbe or virus. It is preferable that the extraction of the calcified substance is carried out without sintering at a high temperature. Herein the high temperature of sintering means any temperature wherein the structure or orientation of calcium carbonate crystal of the calcified substances produced by cells, is affected.

Extracting a calcified substance of the present invention involves washing by resolving protein and other biological molecules attached or associated to the calcified substance with the exposure to detergents such as SDS and others.

### EXAMPLE 1

The present invention is described below with examples, however, the present invention is not to be limited by these examples.

### 1. Material and Methods

### 1) Culture of cell and extraction of calcified substance

UMR-106 cell, an osteoblast-like cell derived from rat osteosarcoma (Partridge, N. C, et al., Cancer Res., 43: 4308 (1983)), was inoculated on a 60 mm diameter culture dish, and cultured in Dulbecco's modified Eagle's medium (DMEM) supplemented with 4.5 g/L of glucose and 10% fetal calf serum in a CO₂ incubator. After reaching to a confluent state, the culture medium was switched to a differentiation medium supplemented with 3% KE-200 (DS Pharma, Osaka), a specific supplement for inducing osteoblastic cell differentiation. Herein, the supplement is comprised of dexamethasone, β (beta)-glycerophosphoric acid and ascorbic acid as the main ingredients. Due to the overconsumption of glucose by the cultured cells, the medium was replaced everyday.

We carried out the studies using cultured human periosteal cells according to the experimental protocol reviewed and approved by the ethical committee for human subject use at Niigata University Medical and Dental Hospital. Human periosteal tissue segments were collected from volunteers after obtaining their informed consents, and incubated in Medium 199 supplemented by 10% of fetal calf serum (herein after referred as a regular growth medium). The initial replacement of culture media was carried out on four-five days, thereafter followed by replacement on about every three days. Human periosteal cells were incubated in a 100 mm dish, and after reaching a confluent state, the cells were subcultured to fresh dishes by trypsin digestion. After three passages, the cells confluent in 100 mm culture dishes were detached and suspended at a density of about 5 x 10⁵ of cells per freezing ampoule according to the conventional freezing methods.

For extracting calcified substances, the human periosteal cells frozen in the ampoule were thawed, inoculated in a 100 mm culture dish, and cultured for four-five days in the normal growth medium. After cells reached a confluent state, the regular growth medium was switched to the differentiation medium, in which was added KE-200 to the regular growth medium, and the cells were further cultured.

As for rat UMR-106 cells, the cells were cultured in a growth medium for two days, and cultured for additional three-five days in a differentiation medium until the cells were collected. Cultured human periosteal cells were cultured in the growth medium for four-five days and further cultured for 23-24 days in the differentiation medium until the cells were collected. The collected cells were dissolved in Laemmli,'s sample buffer for SDS-polyacrylamide gel electrophoresis (Nature, 227: 680-5 (1970)), transferred to 1.5 mL of sample tubes, and boiled at 98°C for 10 min. The insoluble residues after the boiling process were washed three times by centrifugation (2000 rpm for 10 min) and stirring by a voltex mixer in ultrapure water. Further, the ultrapure water was replaced to 70% of aq. ethanol, and the washing procedures were repeated three times by centrifugation and ultrasonic treatment. The insoluble residues suspended in a small amount of 70% aq. ethanol was heated on a heat block at 98°C for 20-30 min, the solvent was evaporated, and finally the calcified substances were prepared from cultured cells. The calcified substance samples were stored in a desiccator at room temperature until used in the experiments described below. At the time just before the addition to the cultured periosteal sheets, about 10 µg (microgram) of the calcified substance sample was suspended in 20 µL (microlitter) of DMEM containing 0.2% of atelocollagen (KOKENCELLGEN, Koken, Tokyo) for each periosteal sheet.

### 2) Observations by microscopy and electron scanning microscopy

To examine how many calcified substances are produced and deposited, UMR-106 cell cultures were fixed and stained with alizarin red. Fixed cells were also observed by an electron scanning microscopy after t-butyl-alcohol freeze-drying. At the same time, the microstructure of the calcified substances extracted from UMR-106 cells was examined by an electron scanning microscopy.

### 3) Analysis by X-ray microanalyzer

Elements contained in the calcified substance sample was quantitatively analyzed by an electron probe microanalyzer (type EPMA-8705, Shimadzu Corp.) by the methods of qualitative analysis and quantitative analysis with standard-less correction. The sample was attached to the stage of X-ray microanalyzer with double-sided conductive carbon tape and deposited with carbon. Accelerated voltage and sample current were 15kV, 0.1 µ (micro)-A, respectively. The region of detection was 100 µm (micrometer) in diameter. The results are expressed as the molar ratio of calcium to phosphorus (Ca/P).

### 4) Fourier transform infrared spectroscopy

Elements contained in the calcified samples were analyzed by the conventional KBr tablet method using a Fourier transform infrared spectrometer type Spectrum One (PerkinElmer Japan).

### 5) X-ray diffraction analysis

X-ray diffraction analysis of the elements was carried out by the conventional powder method using an X-ray diffractometer type miniFlexll (Rigaku Corp.).

### 6) Studies in vitro

Human periosteal segments (2 mm x 2 mm in size) were collected after obtaining of volunteer's informed consents, and placed on porous membranes (VECELL, Asahi Glass Co., Ltd.) for the culture in the regular growth medium. The medium was initially replaced with an interval of four-five days, and subsequently replaced about every three days. After being cultured in the normal growth medium for 13-15 days, the cells migrated out from the periosteal segment and formed a sheet of periosteum. The medium was switched to the differentiation medium containing KE-200 to induce the differentiation of the periosteal sheet. Otherwise, the culture was maintained under the same conditions. The periosteal sheets were further cultured for 7-10 days in the differentiation medium or the regular growth medium, 10 µg (microgram) of the calcified substances suspended in 0.2% atelocollagen solution [20 µL (microlitter)] was added to the central region of the periosteal sheet, which corresponds to the original periosteal tissue segment. For reference, particles of β (beta)-TCP were similarly added to the cultured sheets in a form of suspension in the atelocollagen solution. The periosteal sheets were further cultured for eight-nine days with the calcified substance samples or β (beta)-TCP particles. At the end of culture, the periosteal sheets were subjected to the histological observation, or the in vivo animal implantation studies.

### 7) In vivo animal implantation studies

The cultured periosteal sheet was rinsed 3 times with PBS (-), dissected to a piece (about 5 mm x 5 mm in size), and used for implantation. The dorsal skins of nude mice (Balb/c nu/nu, male, 15-18 gram) were serially cleaned with Povidone-Iodine and aq. ethanol for disinfection, and the surgical procedure was aseptically performed.

The skin was incised by 7-10 mm in length, and the piece of periosteal sheet was implanted into the subcutaneous tissue. The care and use of animals followed the Guiding Principles for the Care and Use of Animals, as approved by Niigata University.

### 8) Histological studies

The periosteal sheet cultured on the porous membrane was directly fixed, dehydrated by series of ethanol, treated with xylene, and embedded in paraffin. The implanted periosteal sheet was retrieved with the surrounding tissues, and embedded into paraffin in the same way. The tissue samples were sectioned and stained with HE, von Kossa, and TRAP (tartarate-resistant acid phosphatase) stainings.

### 2. Results

### 1) Observation of calcified substances derived from cultured cells by microscopy and electron scanning microscopy.

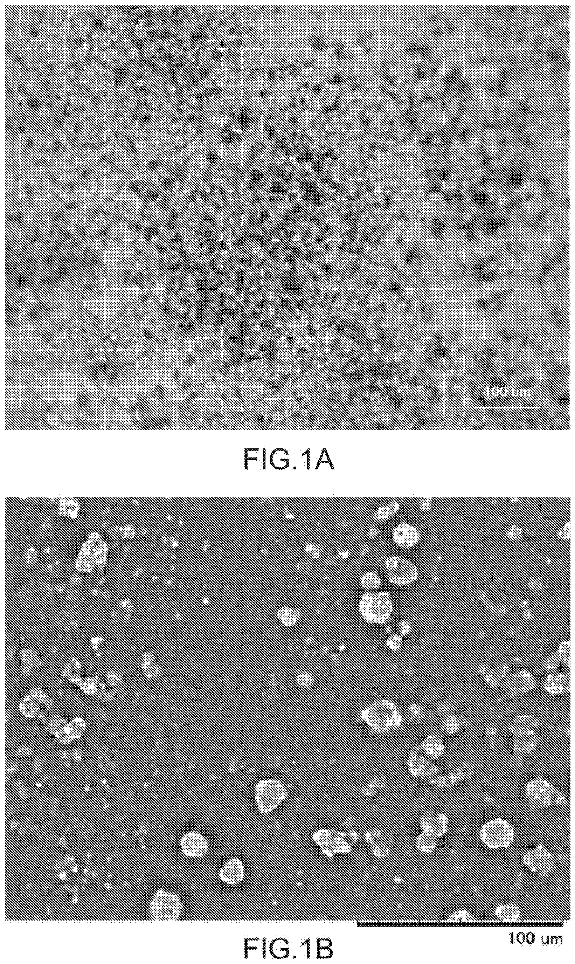

The calcified substances were increasingly deposited by UMR-106 cells over the time of cultivation in the regular growth medium. To obtain greater amounts of calcified substances, however, the medium was switched to the differentiation medium containing the supplements for the induction of osteoblastic cells. Fig. 1 shows a microscopic photograph of UMR-106 cells stained with alizarin red (Fig. 1A) and an electron scanning microscopic photograph (Fig.1B) of calcified substances produced by UMR-106 cells, which were cultured in a culture dish for additional five days after switching to the differentiation medium. Both of the scale bars in Fig. 1A and 1B represent 100 µm (micrometer).

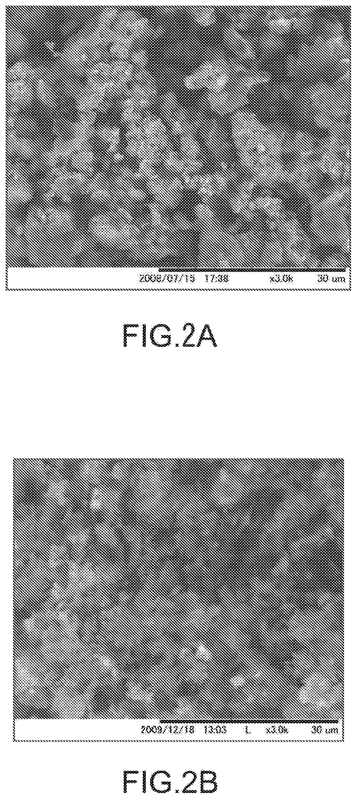

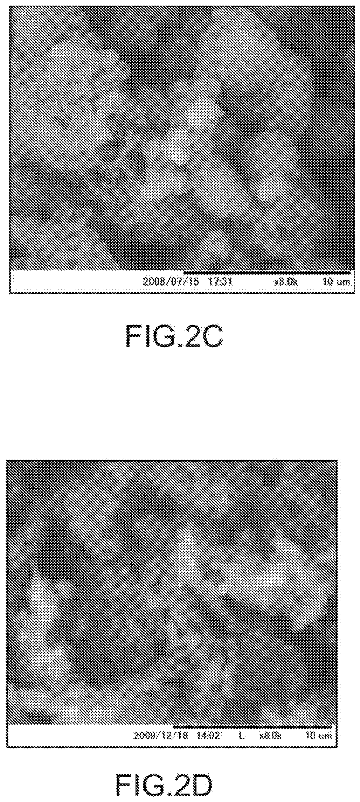

Fig. 2A and 2B shows the electron scanning microscopic photographs with low magnification of the calcified substance extracted from UMR-106 cells and human perioseum cells. UMR-106 cells were cultured in a culture dish for the last five days in the differentiation medium, while human periosteal cells were cultured for the last 24 days in a cultured dish in the differentiation medium. The scale bar in Fig. 2A represents 30 µm (micrometer). Fig. 2C and D show the electron microscopic photographs of the same samples presented in Fig. 2A and B with high magnification. As shown in Fig. 2, calcified substances extracted both from UMR-106 cells and human periosteal cells are characterized by the microstructures that the minimal structural unit was a calcified particle of 1-2 micrometer in diameter, and these units assembled to form micropores of 5-10 µm (micrometer) in diameter. The microstructural characteristic is very similar to that of commercially available porous HAp particles (APACERAM®), a product of HOYA.

### 2) Analysis by X-ray microanalyzer

The amount of calcified substance sample extracted from UMR-106 cells was about 15 mg per one 100 mm dish, while the amount of calcified substance sample extracted from the cultured human periosteal cells was about 3 mg per one 100 mm dish. As well, the amount of extracted calcified substance sample from a piece of mouse femur (ICR, 6 weeks, male) was about 7 mg. The measured Ca/P ratios of calcified substance samples derived from UMR-106 cells and the cultured human periosteal cells were 1.472 and 1.452, respectively. For the comparison, the measured Ca/P ratios of HAp (APACERAM®, HOYA Co. Ltd.) and β (beta)-TCP (OSferion®, OLYMPUS TERUMO BIOMATERIALS CORP.) were 1.832 and 1.681, respectively. As the theoretical values of Ca/P ratio of HAp and-β (beta)-TCP are 1.67 and 1.50 respectively, the measured values were a little higher than the theoretical values. The Ca/P ratios of calcified substance samples extracted from cultured cells were lower than those of HAp and β (beta)-TCP.

### 3) Analysis by Fourier transform infrared spectrophotometry

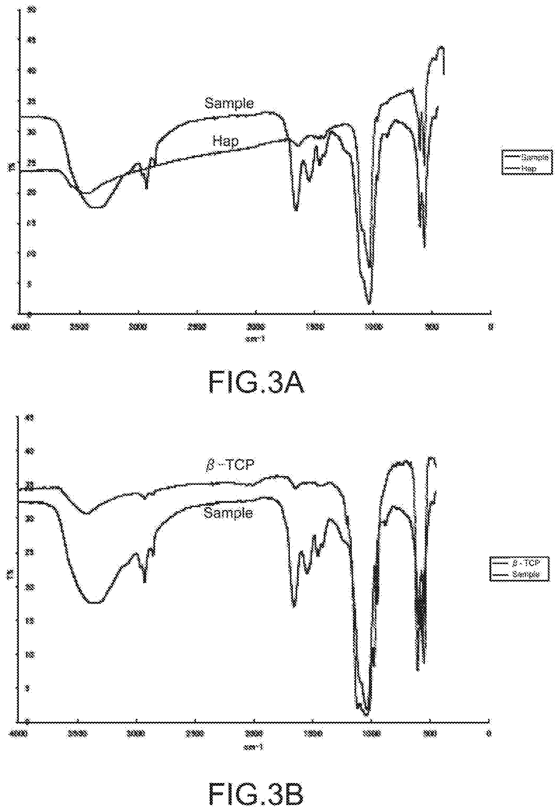

Fig. 3 shows the spectra of Fourier transform infrared spectrophotometry the calcified substance sample extracted from UMR-106 cells, HAp and β (beta)-TCP. In Fig. 3A, the spectra of the calcified substance sample and HAp were matched, and in Fig.3B, the spectra of the calcified substance sample and β (beta)-TCP were matched. It was suggested from Fig. 3A and B that the compositions of calcified substance sample was close to that of HAp rather than that of β (beta)-TCP.

### 4) X-ray diffraction analysis

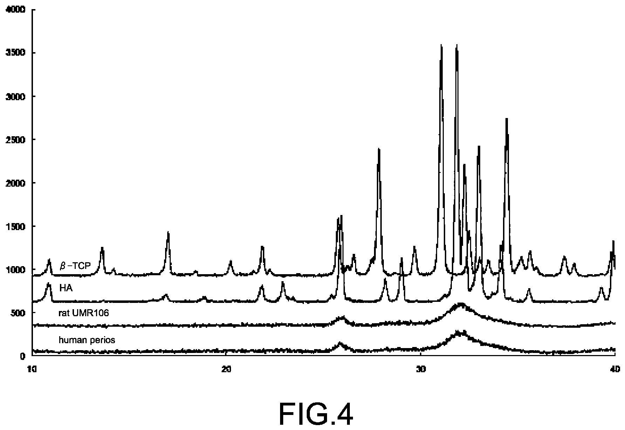

Fig. 4 shows the spectra of X-ray diffraction analysis regarding the calcified substance samples extracted from UMR-106 cells and cultured human periosteal cells, HAp and β (beta)-TCP. The spectra show the diffraction curves of β (beta)-TCP, HAp (HA), the calcified substance sample derived from UMR-106 (rat UMR106) and the calcified substance sample derived from the cultured human periosteal cells (human perios), from the top, respectively. The calcified substance samples derived both from UMR-106 cells and the cultured human periosteal cells were amorphous. However, judged from the point of the angle of maximum peaks, it is suggested that they are of higher similarity to HAp than to β (beta)-TCP. These observations were not conflicting with the results of Fourier transform infrared spectrophotometry. Therefore, with consideration of the results of the said micro X-ray analysis, it was suggested that they might be the types of HAp, in which calcium is more or less defected.

### 5) Cultured periosteal sheet

A cultured human periosteal sheet became capable of producing calcium deposits in response to supplemented differentiation-inducing agents for osteoblastic cells, in the six-week cultivation. This finding implies that spontaneous calcification without aid of such differentiation-inducing agents cannot highly be expected. In the examples of the invention, cultivation was carried out on the porous meshes instead of conventional plastic dishes. However, calcification could not be observed when cultivation was carried out only in the regular growth medium (the figure not shown). Herein below, the cultured sheet incubated only in the growth medium is designated as "undifferentiated periosteal sheet", and the periosteal sheet cultured serially in the growth and the differentiation medium is designated as "differentiation-induced periosteal sheet".

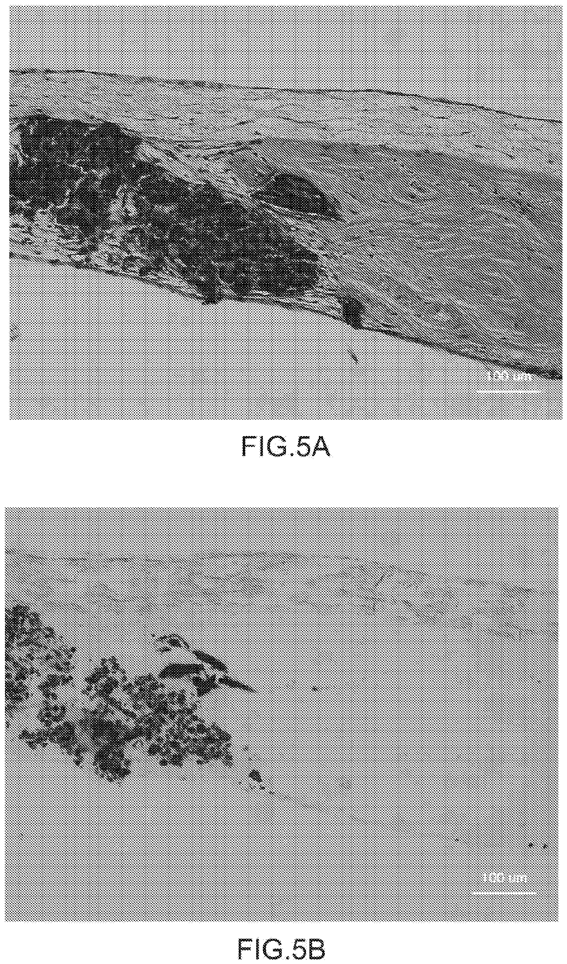

The calcified substance sample derived from UMR-106 cells or the pulverized particles of β (beta)-TCP were added to both of the undifferentiated and differentiation-induced periosteal sheets. Fig. 5A and B shows the microscopic photograph of tissue samples stained with HE (Fig. 5A) and von Kossa stainings (Fig. 5B), respectively, at eight days after addition of calcified substance sample derived from UMR-106 cells to the undifferentiated sheet. As shown in Fig. 5A and B, the calcified substance sample derived from the UMR-106, which were added to the undifferentiated periosteal sheet, were embedded under the surface of multi-layered periosteal sheet, and the original shapes of calcified particle were well reserved at eight days. In the case of the pulverized particles of β (beta)-TCP, the β (beta)-TCP particles were embedded under the surface of multi-layered periosteal sheet, and the original shape was preserved as observed above (the figure not shown).

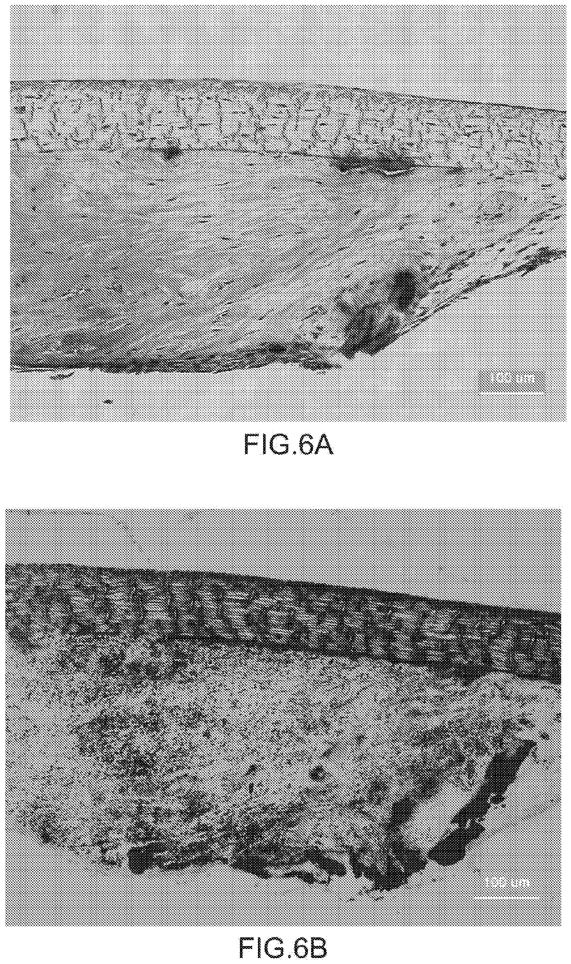

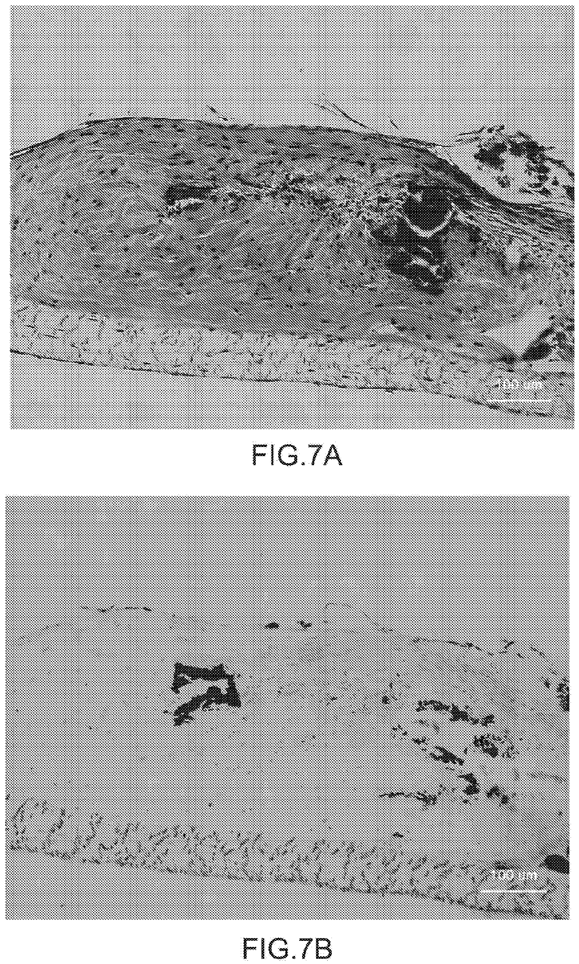

Fig. 6A and B shows the microscopic photographs of tissue samples for HE (Fig. 6A) and von Kossa stainings (Fig. 6B), respectively, at eight days after addition of the calcified substance sample derived from UMR-106 cells to the differentiation-induced periosteal sheet. Fig. 7A and B shows the microscopic photographs of a tissue sample for HE (Fig. 7A) and von Kossa stainings (Fig. 7B), respectively, at eight days after addition of the pulverized β (beta)-TCP particles to the differentiation-induced periosteal sheet. As shown in Fig. 6A and B, when the calcified substance sample derived from UMR-106 were added to the differentiation-induced periosteal sheet, the calcified substance particles was preserved in terms of the shape. Consequently, the newly formed areas positive for von Kossa staining could not be observed. In contrast, in the differentiation-induced periosteal sheet, calcified substances particles were broken into fine particles like powder and concomitantly new calcium deposits were formed widely in the periosteal sheet, which were positive for von Kossa staining. In addition, calcified substances particles were assembled in a part of the differentiation-induced periosteal sheet, and these particles were strongly positive for von Kossa staining. As shown in Fig. 7A and B, when the pulverized particles of β (beta)-TCP were added to the differentiation-induced periosteal sheet, the original shapes of the β (beta)-TCP particles were well preserved and von Kossa-positive.

Although the differentiation-induced periosteal sheet were capable of producing von-Kossa-positive mineral deposits without addition of the calcified substance sample derived from UMR-106 cells, the periosteal sheet produced much greater amounts of mineral deposits when the calcified substance sample was added. This finding indicates that the UMR-106 cell-derived calcified substance sample could once be dissolved and used for cell-dependent mineral deposition in the powder or crust form.

### 6) Studies on implantation into the subcutaneous tissue of nude mice

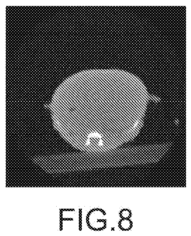

Fig. 8 shows a micro-CT image of a nude mouse one week after the implantation of the differentiation-induced periosteal sheet incubated with the calcified substance sample derived from UMR-106 cells. The gray region depicts the abdominal section of the mouse, the central white structure represents the vertebra, the white band observed at the right marginal region of the abdominal section represents the differentiation-induced periosteal sheet receiving the calcified substance sample derived from UMR-106 cells. As shown in Fig. 8, the differentiation-induced periosteal sheet receiving the calcified substance sample derived from UMR-106 cells, which was implanted in the right side of vertebra, absorbed X-ray clearly one week after implantation, suggesting that the implanted material is highly calcified. In contrast, the undifferentiated periosteal sheet similarly receiving the calcified substance sample derived from UMR-106 cells, which was implanted in the left side of vertebra, remained the calcification at lower levels.

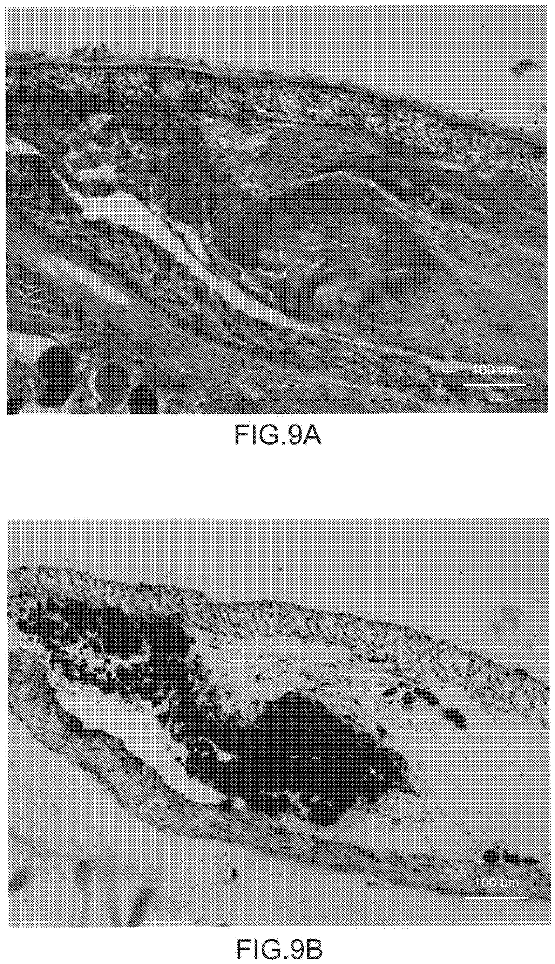

Fig. 9A and B shows the microscopic photographs of a HE (A) and von Kossa (B)-stained sections of a differentiated periosteal cell sheet after fixation, which had been cultured for eight days after supplementing the calcified substance sample extracted from UMR-106 cells and implanted under bilateral back skin region. As shown in Fig. 7A, when the mice receiving the implantation were observed up to 4 weeks, the implanted calcified particles extracted from UMR-106 cells were completely degraded in the central region of the sheet (which corresponds to the periosteal tissue segment originally harvested from periosteal tissue and placed on the porous membrane for the explant culture), and the calcified tissues were newly formed. As shown in Fig. 7B, taken together with the finding that osteoclastic cells were increasingly recruited, it is speculated that the calcified particles extracted from UMR106 cells are initially degraded and absorbed by osteoclasts, and the ionized calcium and phosphates are subsequently applied to calcification by osteoblastic cells.

## Claims

1. A method for producing a base material for regenerating a human bone tissue comprising a calcified substance comprising calcium phosphate extracted from a cultured cell, comprising
a step of extracting a calcified substance from a cultured cell with a calcification capacity,
a step of adding the calcified substance from a cultured cell to a differentiation-induced periosteal sheet, and
a step of incubating the differentiation-induced periosteal sheet with the calcified substance.

2. The method for producing the base material according to claim 1, wherein the cultured cell with a calcification capacity is a mammalian cell.

3. The method for producing the base material according to claim 1 or 2, wherein the cultured cell having a calcification capacity is a cell derived from a hard tissue.

4. The method for producing the base material according to claim 1 or 2, wherein the cultured cell with a calcification capacity is a cell derived from an osteosarcoma cell.

5. The method for producing the base material according to any one of claims 1 to 4, wherein the cultured cell with calcification capacity is a UMR-106 cell.

6. The method for producing the base material according to any one of claims 1 to 5, wherein the step of extracting the said calcified substance comprises extracting without sintering at a high temperature.

7. The method for producing the base material according to claim 6, wherein the step of extracting the said calcified substance comprises extracting by use of a detergent.

## Patentansprüche

1. Verfahren zur Herstellung eines Grundmaterials zur Regeneration von menschlichem Knochengewebe, das eine aus einer kultivierten Zelle extrahierte kalzifizierte Substanz aufweist, die Calciumphosphat enthält, wobei das Verfahren umfasst:
einen Schritt zum Extrahieren einer kalzifizierten Substanz aus einer kultivierten Zelle mit Kalzifizierungsvermögen,
einen Schritt zum Hinzufügen der kalzifizierten Substanz aus einer kultivierten Zelle zu einer differenzierungsinduzierten periostalen Platte, und
einen Schritt zum Inkubieren der differenzierungsinduzierten periostalen Platte mit der kalzifizierten Substanz.

2. Verfahren zur Herstellung eines Grundmaterials nach Anspruch 1, wobei es sich bei der kultivierten Zelle mit Kalzifizierungsvermögen um eine Säugerzelle handelt.

3. Verfahren zur Herstellung eines Grundmaterials nach Anspruch 1 oder 2, wobei es sich bei der kultivierten Zelle mit Kalzifizierungsvermögen um eine aus Hartgewebe erhaltene Zelle handelt.

4. Verfahren zur Herstellung eines Grundmaterials nach Anspruch 1 oder 2, wobei es sich bei der kultivierten Zelle mit Kalzifizierungsvermögen um eine von einer Osteosarkomzelle abgeleitete Zelle handelt.

5. Verfahren zur Herstellung eines Grundmaterials nach einem der Ansprüche 1 bis 4, wobei es sich bei der kultivierten Zelle mit Kalzifizierungsvermögen um eine UMR-106-Zelle handelt.

6. Verfahren zur Herstellung eines Grundmaterials nach einem der Ansprüche 1 bis 5, wobei der Schritt zum Extrahieren der kalzifizierten Substanz ein Extrahieren ohne Sintern bei einer hohen Temperatur umfasst.

7. Verfahren zur Herstellung eines Grundmaterials nach Anspruch 6, wobei der Schritt zum Extrahieren der kalzifizierten Substanz ein Extrahieren unter Verwendung eines Detergens umfasst.

## Revendications

1. Procédé de production d'un matériau de base pour régénérer un tissu osseux humain comprenant une substance calcifiée comprenant du phosphate de calcium extrait d'une cellule en culture, comprenant
une étape d'extraction d'une substance calcifiée d'une cellule en culture ayant une capacité de calcification,
une étape d'ajout de la substance calcifiée à partir d'une cellule en culture dans une plaque de périoste induite par différenciation, et une étape d'incubation de la plaque de périoste induite par différenciation avec la substance calcifiée.

2. Procédé de production du matériau de base selon la revendication 1, dans lequel la cellule en culture ayant une capacité de calcification est une cellule de mammifère.

3. Procédé de production du matériau de base selon la revendication 1 ou 2, dans lequel la cellule en culture ayant une capacité de calcification est une cellule provenant d'un tissu dur.

4. Procédé de production du matériau de base selon la revendication 1 ou 2, dans lequel la cellule en culture ayant une capacité de calcification est une cellule provenant d'une cellule d'ostéosarcome.

5. Procédé de production du matériau de base selon l'une quelconque des revendications 1 à 4, dans lequel la cellule en culture ayant une capacité de calcification est une cellule UMR-106.

6. Procédé de production du matériau de base selon l'une quelconque des revendications 1 à 5, dans lequel l'étape d'extraction de ladite substance calcifiée comprend l'extraction sans frittage à haute température.

7. Procédé de production du matériau de base selon la revendication 6, dans lequel ladite étape d'extraction de ladite substance calcifiée comprend l'extraction à l'aide d'un détergent.
